(19) Europäisches Patentamt / European Patent Office / Office européen des brevets

(11) **EP 4 299 698 A1**

(12) **EUROPEAN PATENT APPLICATION**

(43) Date of publication:
**03.01.2024 Bulletin 2024/01**

(21) Application number: **22182034.3**

(22) Date of filing: **29.06.2022**

(51) International Patent Classification (IPC):
*C11D 1/83* $^{(2006.01)}$     *C11D 3/395* $^{(2006.01)}$
*C11D 3/48* $^{(2006.01)}$     *C11D 11/00* $^{(2006.01)}$
*C11D 1/12* $^{(2006.01)}$     *C11D 1/66* $^{(2006.01)}$
*C11D 1/75* $^{(2006.01)}$     *A61L 2/18* $^{(2006.01)}$

(52) Cooperative Patent Classification (CPC):
**C11D 3/48; A61L 2/186; C11D 1/83; C11D 3/3951;
C11D 3/3956; C11D 11/0064;** C11D 1/12;
C11D 1/662; C11D 1/75

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **G1-BlueTec GmbH
20149 Hamburg (DE)**

(72) Inventor: **ECKHOFF, Heinrich
20149 Hamburg (DE)**

(74) Representative: **Uexküll & Stolberg
Partnerschaft von
Patent- und Rechtsanwälten mbB
Beselerstraße 4
22607 Hamburg (DE)**

(54) **COMPOSITIONS COMPRISING SURFACTANTS**

(57)     The present invention provides compositions comprising water, an amine oxide surfactant and at least two further surfactants, wherein the composition has a pH in the range of 3 to 5.8 and wherein at least one surfactant of the at least two further surfactants is an anionic surfactant at the pH of the composition and the amount (w/w) of the anionic surfactants in total exceeds the amount of the amine oxide surfactants by at least 4.1 times.

EP 4 299 698 A1

**Description**

The present invention relates to compositions of surfactants, in aqueous solutions, which have a very low surface tension in the acidic pH range. The combination of the effects of an acidic environment and the lowest possible surface tension of aqueous solutions can support a variety of active ingredients in their application. For example, not only biocidal or phytoprotective substances can be considered as active substances to be supported for the treatment of surfaces, but also explicitly other surfactants with special properties and surface tension in the acidic pH range that can be improved.

Surfactants supporting a wide variety of active ingredients are well known in the state of the art and range from printing inks to crop protection products and vaccines. Consequently, the constant attempt to synergistically improve the surfactant performance of the (overall) formulation through special surfactant compositions is not only driven by the desire for better washing and cleaning performance. In simple terms, almost all surfactant mixtures represent an attempt to formulate further advantageous surfactant properties on the basis of the lowest possible surface tension. In this context, corresponding surfactant performances in the acidic pH range are of particular interest. The acidic pH range not only leads to better accessibility of otherwise hidden structures and an independent pH effect on microorganisms via the lime-dissolving effect of the acid(s), but it also represents a milieu for many non-surfactant active ingredients in which they find their optimum effect and/or can be made (storage) stable.

There is therefore a need for further improved (base) surfactant formulations having an acidic pH range, which, even with a small proportion of the application formulation, are able to dominantly determine a low surface tension in the (application) overall systems and are also relatively stable with regard to their surface tension against a drop in concentration, as can occur as a result of large micro-surfaces of porous-structured surfaces (possibly also due to dirt and/or germs).

Surprisingly, it has now been found that this problem can be solved if an aqueous composition comprises at least one amine oxide surfactant and at least two further surfactants, the composition having a pH value in the range from 3.0 to 5.8 and wherein at least one surfactant of the at least two further surfactants is an anionic surfactant at the pH of the composition and the amount (w/w) of the anionic surfactants in total exceeds the amount of the amine oxide surfactants by at least 4.1 times. Corresponding compositions maintain a very low surface tension even in combination with further surfactants and other active ingredients of washing and/or sterilizing compositions, such as hydrogen peroxide. Even if the surfactant concentration of the composition is experimentally reduced by, for example, 50% compared to the application solution.

In one embodiment, the present invention provides compositions comprising at least one amine oxide surfactant and at least two other surfactants that are anionic in the pH range, but the composition omits the anionic surfactants sodium lauryl ether sulphate (SLES, CAS: 68585-34-2) and/or alkyl benzene sulphonate, such as secondary alkane sulphonate (SAS, CAS: 68188-18-1).

While the amine oxide surfactants were known to have excellent grease dissolving and surface tension reducing effects, they were also known to be particularly difficult to combine with anionic surfactants in the pH range below 5.8.

Corresponding compositions exhibit very low surface tension, even when combined to a substantial extent with surfactants which alone are characterized by comparatively poor surface tension, such as: SDS (sodium dodecyl sulphate, CAS: 151-21-3) and/or fatty alcohol glucosides (APG) and/or SAS and/or SLES. This also opens up the possibility of formulating surfactant compositions based mainly on SDS/SLES and APG in such a way that they achieve surface tension values < 27 mN/m in the acidic range. In this context, although it was known that the relatively mild amine oxide surfactants have excellent fat dissolving and surface tension lowering effects, it was also known that it is particularly difficult to combine them with anionic surfactants in the pH range below 5.8.

Surfactants are identified in the context of the present invention as non-ionic, ionic, anionic or cationic surfactants if the compound is uncharged, negatively charged or positively charged at the pH of the composition. The quantities of the surfactants refer in each case to the active substance. All concentrations are given relative to the total weight of the aqueous liquid composition.

In a preferred aspect, the compositions of the present invention comprise:

(a) the at least one amine oxide surfactant is present at a concentration in the range of 0.05 to 1.2% (w/w), preferably in the range of 0.05 to 1% (w/w), most preferably in the range of 0.1 to 0.8% (w/w); and
(b) the at least two anionic surfactants are together present in a concentration in the range from 0.1 to 3.8% (w/w), preferably in the range from 0.15 to 2.8% (w/w), most preferably in the range of from 0.2 to 2.4% (w/w).
In the context of the present invention, all concentrations given in % refer to the % w/w in relation to the total weight of the composition.

The pH of the compositions according to the invention is in the range of 3 to 5.8, preferably in the range of 3 to 5.6, most preferably in the range of 3 to 5.2 and most preferably in the range of 3 to 4.4.

In one aspect, the compositions according to the invention further comprise at least one surfactant which is a nonionic surfactant at the pH of the composition but not selected from the group of N-acylated amino acid surfactants and/or N-acylated peptide surfactants and is further characterized in that the weight ratio (w/w) of this so defined nonionic surfactant group to the anionic surfactants is in the range of 1:4.4 to 4.4:1.

In particularly preferred embodiments, the compositions of the present invention are further characterized in that

(a) the weight ratio (w/w) of the at least one amine oxide surfactant to the sum of the anionic surfactants is in the range from 1:12 to 1:4.1, preferably in the range from 1:9 to 1:4.1, more preferably in the range from 1:8 to 1:4.5, most preferably in the range from 1:7 to 1:5; or
(b) the weight ratio (w/w) of the at least one amine oxide surfactant, when a SLES surfactant and/or a SDS surfactant is present in the composition, is in the range of (amine oxide surfactants):(SLES + SDS) = 1:2.9 to 1:0.4.

The present invention therefore provides amongst others the following compositions:

(A) Composition comprising water and:

(a) an amine oxide surfactant in a concentration of 0.05 to 1.2% (w/w), and
(b) SLES, SDS, SAS, SFA-P or PL-C or a mixture thereof in a concentration of 0.15 to 3.8% (w/w); and
(c) APG in a concentration of 0.8 to 4% (w/w);
wherein the composition has a pH value in the range of 3.0 to 5.and wherein the amount (w/w) of the surfactants of (b) in total exceeds the amount of the amine oxide surfactants of (a) by at least 4.1 times.

(B) Composition comprising water and:

(a) an amine oxide surfactant in a concentration of 0.05 to 1.2% (w/w), and
(b) SLES, SDS, SAS, SFA-P or PL-C or a mixture thereof in a concentration of 0.15 to 3.8% (w/w); and
(c) APG in a concentration of 0.8 to 4% (w/w); and
(d) $H_2O_2$ in a concentration of 0.2 to 4% (w/w); and

wherein the composition has a pH value in the range of 3.0 to 5 and wherein the amount (w/w) of the surfactants of (b) in total exceeds the amount of the amine oxide surfactants of (a) by at least 4.1 times.

(C) Composition comprising water and:

(a) an amine oxide surfactant in a concentration of 0.05 to 1.2% (w/w), and
(b) SDS, and SFA-P and PL-C in a concentration of 0.8 to 3% (w/w); and
(c) APG in a concentration of 0.8 to 4% (w/w);
(d) calcium/potassium sulphite, preferably in a concentration of 0.005 to 0.35% (w/w);

wherein the composition has a pH value in the range of 3.0 to 5 and wherein
the amount (w/w) of the surfactants of (b) in total exceeds the amount of the amine oxide surfactants of (a) by at least 4.1 times.

(D) Composition comprising water and:

(a) an amine oxide surfactant in a concentration of 0.05 to 1.2% (w/w), and
(b) SDS, and SFA-P and PL-C in a concentration of 0.8 to 3% (w/w); and
(c) APG in a concentration of 0.8 to 4% (w/w);
(d) $H_2O_2$ in a concentration of 0.2 to 4% (w/w); and
wherein the composition has a pH value in the range from 3.0 to 5 and wherein the amount (w/w) of the surfactants of (b) in total exceeds the amount of the amine oxide surfactants of (a) by at least 4.1 times.

(E) Composition comprising water and:

(a) an amine oxide surfactant in a concentration of 0.05 to 1.2% (w/w), and
(b) SDS, and SFA-P and PL-C in a concentration of 0.8 to 3% (w/w); and
(c) APG in a concentration of 0.8 to 4% (w/w);
(d) $H_2O_2$ in a concentration of 0.2 to 4% (w/w); and

(e) one or more C12 to C14 fatty acid sulphonates in a concentration of 0.01 to 0.9% (w/w) and/or one or more phosphorus ester-based surfactants with a chain length of C8 to C18, in the concentration of 0.008 to 2.4% (w/w), and/or one or more surfactants selected from the group of N-acylated amino acid surfactants and/or N-acylated peptide surfactants having an anionic effect at the pH of the solution, in a concentration of 0.01 to 0.09% (w/w); and wherein the composition has a pH value in the range from 3.0 to 5 and wherein the amount (w/w) of the surfactants of (b) in total exceeds the amount of the amine oxide surfactants of (a) by at least 4.1 times.

In another particularly preferred aspect the compositions according to the invention contain one or more surfactants selected from the group of N-acylated amino acid surfactants and/or N-acylated peptide surfactants having an anionic effect at the pH of the solution, in a concentration of 0.01 to 0.09% (w/w) and one or more C12 to C14 fatty acid sulphonates in a concentration of 0.01 to 0.9% (w/w).

In a further embodiment, the composition according to the invention comprises one or more C12 to C14 fatty acid sulphonates in a concentration of 0.01 to 0.9% (w/w) and/or one or more phosphorus ester-based surfactants with a chain length of C8 to C18, in the concentration of 0.008 to 6.2% (w/w), and/or one or more surfactants selected from the group of N-acylated amino acid surfactants and/or N-acylated peptide surfactants having an anionic effect at the pH of the solution, in a concentration of 0.01 to 0.9% (w/w).

Compositions according to the invention may comprise further surfactants which are cationic, anionic or non-ionic in the pH range. In particular, the compositions may comprise surfactants containing a sulphate or sulphonate group, preferably selected from the group consisting of methyl ester sulphonates (MES) and alkenyl carboxy sulphonates (ACS) in a concentration of 0.01 to 0.9% (w/w) and/or alkyl polyglycosides (APG), as in the form of the commercially available products: Glucopon 215 or Plantacare 818, in the concentration of 0.20 to 2.80% (w/w) and/or cocamidopropyl betaine, as in the form of the commercially available product BETADET-HR, in a concentration of 0.20 to 2.50% (w/w).

The compositions according to the invention may comprise numerous other components, including:

(a) hydrogen peroxide in a concentration of 0.01 to 8.5% (w/w), preferably 0.2 to 6.0% (w/w), more preferably 0.3 to 4% (w/w);

(b) one or more acids in a total concentration of 0.01 to 1.5% (w/w) selected from glycolic acid, fumaric acid, benzoic acid, salicylic acid, acetylsalicylic acid, citric acid, oxalic acid, succinic acid, formic acid, lactic acid, methanesulfonic acid and amidosulfonic acid;

(c) one or more alcohols in a total concentration of 0.2 to 49% (w/w), preferably 0.3 to 28% (w/w), the alcohol preferably being selected from ethanol and 2-propanol;

(d) one or more fatty acids, preferably fatty acids other than a fatty acid used as a base of a sulphonated surfactant in the composition, preferably at a concentration of 0.1 to 6% (w/w);

(e) one or more lipids, such as sophorolipids, for example in the form of the sophorolipid-based commercial product Break-Thru SF 420, preferably in a concentration of 0.002 to 3% (w/w) or rhamnolipids, such as rhamnolipids, identified as CAS No: 4348-76-9, or products based on this base, preferably in a concentration of 0.01-3% (w/w);

(f) plant extracts and/or their synthetic equivalents, such as, inter alia, D-limonene ((R)-(+)-limonene) (CAS 5989-27-5), preferably in a concentration of 0,002 to 6% (w/w); geranial (CAS: 141-27-5), neral (CAS: 106-26-3) and the mixture thereof, citral (CAS: 5392-40-5); citronella oil;

(g) one or more diols, such as in particular 3-methyl-1,3-butanediol (CAS: 2568-33-4), preferably in a concentration of 0,2 to 15% (w/w);

(h) zwitterionic amino acids, such as in particular proline and hydroxyproline, preferably in a concentration of 0.01 to 6% (w/w);

(i) sodium diisopropylnaphthalene sulphonate, preferably in a concentration of 0,01 to 1,2% (w/w);

(j) alkenyl amber anhydride, preferably in a concentration of 0,01 to 6% (w/w);

(k) calcium/potassium sulphite (or sulphite ions and/or sulphur dioxide), preferably in a concentration (or concentration equivalent) of 0.005 to 0.35% (w/w);

(l) perfumes, complexing agents, thickeners

(m) peracetic acid (0.005 to 3.0 ppm) and/or chlorine dioxide (0.01 to 12 ppm)

(n) hypochlorous acid (0.05 to 6 ppm) and/or chlorine dioxide (0.01 to 12 ppm)

(o) mixtures of (a) to (n).

As indicated above, a key finding of the present invention is that compositions containing at least three surfactants can be prepared which still have a surface tension in the acidic pH range ($\leq$ 5.8 pH) of less than 28 mN/m, even after the addition of substantial amounts of APG. To determine the exact surface tension values, a tensiometer was first adjusted to 22.5 mN/m with ethanol (99.8%) and then the value was obtained as the mean value of 3 measurements.

In preferred embodiments, the application compositions comprise at least four surfactants and are characterized in that they exhibit a surface tension after 30 seconds of less than 27.5 mN/m, preferably of less than 26.5 mN/m or less than 25.5 mN/m, at an APG content in the solution of > 0.3 % in the acidic pH range.

In another embodiment, the invention comprises compositions containing no ethoxylated compounds or containing them only in a relatively small proportion of the total surfactant amount in the application solution of 10 to 35% (w/w), preferably of 6 to 25% (w/w) and more preferably of 0.5% to 0% (w/w).

The compositions of the present invention may be prepared using numerous different processes. However, in a preferred aspect, the compositions of the invention are obtainable by a process comprising the steps of adding the amine oxide surfactant:

(a) onto the solvent prior to the addition of one or more anionic surfactants;
(b) within 60 seconds prior to the addition of the one or more anionic surfactants to the solvent; or
(c) simultaneously with the addition of the one or more anionic surfactants to the solvent.

In particular, the compositions according to the invention can be used for cleaning and/or sterilizing surfaces and devices. Accordingly, the present invention also provides methods for applying a composition as described above to a surface for cleaning or sterilizing the same, the method comprising steps wherein the composition is mixed from at least two compartments at least 21 days before use and at the latest during application and the pH of the composition differs from the pH of at least one component by at least 0.5 to 6, preferably by 1 to 5, most preferably 1 to 2.

The present invention further provides methods for sterilizing a surface, the method comprising steps wherein a composition as described above is applied to a surface and wherein the application achieves a reduction in sporicidal activity of *Clostridioides difficile* of at least log 2 in a 4-field test after 5 minutes, preferably at least log 3 or at least log 4 in a 4-field test after 5 minutes, more preferably at least 4 log levels after 4 minutes or after 2 minutes, wherein the 4-field test is performed according to EN 16615.

The following abbreviations are used in the following examples and throughout this application:

APG: C8-16 fatty alcohol glucoside, a non-ionic surfactant, for example Plantacare® 818 UP commercially available from BASF; CAS numbers: 141464-42-87732-18-568515-73-1110615-47-9.

AO: Amine oxide or alkyl dimethylamine oxide, a cationic surfactant, for example Libranox AO 1214 commercially available from Libra Speciality Chemicals Ltd, CAS-No30806228-4.

GA: Glycolic acid; CAS number 79-14-1.

SAS: sodium alkyl sulfate, an anionic surfactant, such as Mersolat H 40% commercially available from Lanxess; CAS number 68188-18-1.

SDS: sodium dodecyl sulfate or 2-(2-dodecyloxyethoxy)ethyl sulphate, an anionic surfactant, CAS number: 3088-31-1 / 9004-82-4 / 68891-38-3 / 1335-72-4 / 68585-34-2 / 91648-56-5.

SFA: C12 to C14 fatty acid sulfonate, an anionic surfactant, for example Texapon SFA UP powder commercially available from BASF, mixture of primary secondary sodium alkyl sulfonates with an average chain length of C15.

SLES: sodium laureth sulfate, an anionic surfactant.

PL-C: Perlastan C, acidic form of anionic surfactant in the acidic pH range between 5.8 and about 3, commercially available from Schill+Seilacher, INCI name: Cocoyl Sarcosine; CAS number 68411-97-2.

EXAMPLE:

Application formulation of an aqueous solution

$$A1 = 1.20 \text{ \% SLES} + 0.30 \text{ \% AO}$$

$$B1 = 1.20 \text{ \% APG}$$

Compositions were produced by mixing in deionized water.

The dynamic surface tension of the following samples was determined using a p-Resur tensiometer, for example from BPT Mobilie Krüss. In table 1 MPS indicates the measurement point, which is the number of seconds after beginning of the measurement in the tensiometer. The samples tested represent different combinations of the above base formulation (A1 + B1) and different supplements (SAS, PL-C or SFA-P). The concentration of the second anionic surfactant in each case was determined at the optimum from the parameters reduction of surface tension and stability of the transparent microemulsion. Surprisingly, different concentrations were found for the second anionic surfactant, but the surface tension optima were very close to each other. Insofar as Table 1 indicates a concentration of less than 100%, the composition tested is the composition from the corresponding 100% line which was diluted in deionized water.

**Table 1**

| A1 + B1 plus | Conc. | MP1-OFS 0.4 sec. | MP2-OFS 2.5 sec. | MP3-OFS 8.5 sec. | MP4-OFS 30 sec. | pH | Tensiometer calibrated with |
|---|---|---|---|---|---|---|---|
| 0.30 % SAS | 100 % | 33.72 | 29,52 | 26.56 | 25.78 | 4,04 | Ethanol (99.8 %) / 22.5 mN/m |
| 0.30 % SAS | 100 % | 31.00 | 26.15 | 24.85 | 24.24 | 4.04 | $H_2O$ (100 % %) / 72.5 mN/m |
| 0.30 % SAS | 50 % | 33.30 | 26.97 | 25.07 | 24.09 | | Ethanol (99.8 %) / 22.5 mN/m |
| 0.30 % SAS | 50 % | 32.06 | 26.03 | 24.33 | 23.35 | | $H_2O$ (100 % %) / 72.5 mN/m |
| 0.30 % SAS | 25 % | 36.84 | 28.46 | 26.08 | 24.55 | | |
| 0.09 % PL-C | 100 % | 33.58 | 28.26 | 26.61 | 25,77 | 4.06 | Ethanol (99.8 %) / 22.5 mN/m |
| 0.09 % PL-C | 100 % | 31.24 | 26.31 | 24.94 | 24.21 | 4.06 | $H_2O$ (100 %) / 72.5 mN/m |
| 0.09 % PL-C | 50 % | 33.40 | 27.09 | 25.25 | 24.20 | | Ethanol (99.8 %) / 22.5 mN/m |
| 0.09 % PL-C | 50 % | 32.02 | 26.21 | 24.52 | 23.49 | | $H_2O$ (100 %) / 2.5 mN/m |
| 0.09 % PL-C | 25 % | 36.93 | 28.67 | 26.29 | 24.77 | | |
| 0.20 % SFA-P | 100 % | 33.67 | 28,21 | 26,46 | 25.62 | 4.04 | Ethanol (99.8 %) / 22.5 mN/m |
| 0.20 % SFA-P | 100 % | 31.19 | 26.28 | 24.95 | 24.09 | 4.04 | $H_2O$ (100 %) / 72.5 mN/m |
| 0.20 % SFA-P | 50 % | 33.44 | 27.06 | 25.11 | 24.00 | | Ethanol (99.8 %) / 22.5 mN/m |
| 0.20 % SFA-P | 50 % | 32.19 | 26.18 | 24.46 | 23.34 | | $H_2O$ (100 %) / 72.5 mN/m |
| 0.20 % SFA-P | 25 % | 37.59 | 29.21 | 26.59 | 25.06 | | |

The effect of large (porous) surfaces (possibly also due to dirt and germs) was simulated to some extent by diluting the existing aqueous composition by 50% with deionized water. This is based on the consideration that the surface-active surfactants concentrate more on the boundary surfaces than in the solution and thus, in the case of relatively large surfaces, a decreasing surfactant concentration can occur during application compared to the initial solution with measuring vessel. In this context, it is considered quite advantageous if the surface tension does not increase significantly by a 50% dilution, remains constant or even decreases further. These effects are denoted by Δ in Table 2.

The values shown in Table 2 below were measured as the values in Table 1, above, and subsequently weighted as follows:

A = measured value of SFO in mN/m and average of three measurements (when the meter is set with ethanol 99.8%).
B = measured value of SFO in mN/m and average of three measurements (when the meter is set with demineralized water).

$$MP1: (A \times 1) + (B \times 1) / 2$$

$$MP2: (A \times 2) + (B \times 2) / 3$$

$$MP3: (A \times 3) + (B \times 1) / 4$$

$$MP4: (A \times 4) + (B \times 1) / 5$$

Table 2

| Formulation A1 + B1 plus | Conc. | mN/m 0.4 sec. | Δ | mN/m 2.5 sec. | Δ | mN/m 8.5 sec. | Δ | mN/m 30 sec. | Δ | pH |
|---|---|---|---|---|---|---|---|---|---|---|
| 0.30 % **SAS** | 100 % | 32.36 | | 28.40 | | 26.13 | | **25.47** | | 4,04 |
| diluted with deionized water | 50 % | 32.68 | + 0.32 | 26.66 | -1.74 | 24,89 | -1.24 | 23.94 | -1.53 | |
| | | | | | | | | | | |
| 0.09 % **PL-C** | 100 % | 32.41 | | 27.61 | | 26.19 | | **25,46** | | 4.06 |
| diluted with deionized water | 50 % | 32.71 | + 0.30 | 26.80 | -0.81 | 25.07 | -0,81 | 24.01 | -1.45 | |
| | | | | | | | | | | |
| 0.20 % **SFA-P** | 100 % | 32.43 | | 27.57 | | 26,08 | | **25.30** | | 4.04 |
| diluted with deionized water | 50 % | 32.82 | + 0.39 | 26.77 | -0.80 | 24.95 | -1.13 | 23.87 | -1.43 | |

Results:

The three variable components did not lead to a deterioration of the solution, but rather to a slight but uniform improvement of the dynamic surface tension and an improvement of its stability. Including two of the variable components (SAS; PL-C; SFA-P) resulted in a composition sufficiently stable for an efficient stock production, i.e. maintaining stability over a period of 2 to 6 weeks. Introducing all three of the variable components (SAS; PL-C; and SFA-P) resulted in a fully stable composition maintaining stability for ≥ 6-months, even in lower temperature ranges around 15°C. Samples of this composition became cloudy even after 7-day storage at 4°C, but reorganized themselves at room temperature to transparent microemulsions with comparable surface tension values. This means that the prerequisites for use, for example, with a biocidal active ingredient in the field of disinfectants and within the scope of common practice are also fulfilled.

The anti-microbial activity of the following compositions was determined by means of desquantitative 4-field tests for the assessment of bactericidal activity on non-porous surfaces with mechanical action using wipes in the medical field (4-field test; phase 2, step 2) / based on prWI - sporicidal activity on non-porous surfaces 4-field test (April 2019), EN 16615 (2015).

4-Field Analysis, 2 min / EN 16615 / C. difficile

**Table 3**

| Sample | H₂O₂ | SLES | AO | SFA-P | APG | SAS | PL-C | GA | pH | EWZ | log-RF |
|--------|------|------|-----|--------|------|------|------|------|------|------|--------|
| 1 | 2 | 1.20 % | 0.36 % | ------- | 0,30 % | 0.36 % | --- | 0.15 % | 3,96 | 2,0 min | 3,27 |
| 2 | 2 | 1.20 % | 0.36 % | ------- | 0,60 % | 0.36 % | --- | 0.15 % | 4,01 | 2,0 min | 4,36 |
|  | 2 |  |  |  |  |  |  |  |  |  |  |
| 3 | 2 | 1.20 % | 0.36 % | 0,25 | 0,30 % | 0.36 % | --- | 0.15 % | 3,97 | 2,0 min | 4,54 |
| 4 | 2 | 1.20 % | 0.36 % | 0,25 | 0,30 % | 0.36 % | 0,0 9 % | 0.15 % | 4,04 | 2,0 min | 4,54 |

(pH was adjusted with methanesulfonic acid (CAS number: 75-75-2) and sodium hydroxide (CAS: 1310-73-2)).

Results:

First, the long-term storage-stable sample 4 shows that it did reduce at a moderate pH of 4.04 and without addition of one or more phosphorus surfactants, with 2% $H_2O_2$, C. *diff.* spores by more than 4 log levels in 2 minutes. Although the relatively small amount of 0.09% PL-C significantly improves the stability compared to sample 1, the addition of this compound - at least at the concentration chosen here and at the performance level tested - does not lead to any deterioration in the performance of the adjuvant system for $H_2O_2$ as a disinfectant.

Furthermore, a comparison of sample 1 with sample 3 shows that an increase in APG of the order of 0.30% improves the microbial efficacy against C. *diff.* in the 4-field test by more than one log level.

According to the teaching of the present invention it is also possible to formulate high-performance surfactant compositions in the acidic pH range that completely dispense with ethoxylated compounds. As an example, the following aqueous composition was formulated with a pH of 4.02 and the surface tension was determined according to the method described above:

0.32% AO + 0.7% SDS + 0.7% SFA-P + 0.09% PL-C + 2.95% APG + 0.2 % sulphite: OFS (30 sec.) = 25.75 mN/m

The more than 4-fold concentration of APG compared to SDS, especially in combination with AO, is able to completely compensate for the otherwise undesirable degree of aggressiveness of SDS towards certain surfaces.

**Claims**

1.  A composition comprising water, an amine oxide surfactant and at least two further surfactants, wherein the composition has a pH in the range of 3 to 5.8 and wherein at least one surfactant of the at least two further surfactants is an anionic surfactant at the pH of the composition and the amount (w/w) of the anionic surfactants in total exceeds the amount of the amine oxide surfactants by at least 4.1 times.

2.  A composition according to claim 1, wherein

    (a) the at least one amine oxide surfactant is present at a concentration in the range of 0.05 to 1.2% (w/w), preferably in the range of 0.05 to 1% (w/w), most preferably in the range of 0.1 to 0.8% (w/w); and
    (b) the concentration of the at least one anionic surfactant in the composition is in the range of 0.15 to 3.8% (w/w), preferably in the range of 0.3 to 2.8% (w/w) or 0.4 to 2.6% (w/w), most preferably in the range of 0.8 to 2.4% (w/w).

3. The composition of claim 1, wherein the composition has a pH in the range of 3.0 to 5.6, preferably in the range of 3.0 to 5.5, most preferably in the range of 3.5 to 5.2, and most preferably in the range of 3.9 to 4.4.

4. A composition according to any one of claims 1 to 3, further comprising at least one surfactant which is a non-ionic surfactant at the pH of the composition.

5. A composition according to any one of claims 1 to 4, wherein

   (a) the weight ratio (w/w) of the at least one amine oxide surfactant to the sum of the anionic surfactants is in the range from 1:12 to 1:4.1 preferably in the range from 1:10 to 1:4.1, more preferably in the range of 1:9 to 1:4.1, most preferably in the range from 1:7 to 1:5; or
   (b) the weight ratio (w/w) of the at least one amine oxide surfactant to the SLES surfactant is in the range of 1:12 to 1:1, preferably in the range of 1:9 to 1:1.2, more preferably in the range of 1:9 to 1:2, most preferably in the range of 1:3.9 to 1:2.2.

6. Composition according to any one of claims 1 to 5, wherein the weight ratio (w/w) of nonionic surfactants to the anionic surfactants ranges from 1:4.4 to 4.4:1.

7. The composition according to any one of claims 1 to 6, wherein the composition comprises at least three surfactants containing a sulfate or sulfonate group, preferably selected from the group consisting of sodium dodecyl sulfate (SDS, CAS:151-21-3), sodium lauryl ether sulfate (SLES, CAS: 68585-34-2), secondary alkane sulfonate (SAS, CAS: 68188-18-1), sulphonated fatty acids (SFA, such as disodium 2-sulfolaurate), methyl ester sulphonates (MES) and alkenyl carboxy sulphonates (ACS), wherein the relative concentration of surfactants comprising a sulphate or sulphonate group of the sum of anionic surfactants is more than 5%, preferably more than 8% and in particular more than 12%.

8. The composition according to any one of claims 1 to 7, wherein the composition does not comprise one or more of sodium lauryl ether sulfate (SLES, CAS: 68585-34-2), secondary alkane sulfonate (SAS, CAS: 68188-18-1) and cocamidopropyl betaine.

9. A composition according to any one of claims 1 to 8, the composition further comprising, all concentrations being relative to the total weight of the aqueous liquid composition:

   (a) hydrogen peroxide in a concentration of 0.01 to 8.5% (w/w), preferably 0.2 to 6.0% (w/w), more preferably 0.3 to 4% (w/w);
   (b) one or more C12 to C14 fatty acid sulphonates in a concentration of 0.01 to 0.9% w/w;
   (c) one or more C12 to C14 fatty acid sulphonates in a concentration of 0.01 to 0.9 w/w and hydrogen peroxide in a concentration of 0.01 to 5.0% (w/w);
   (d) one or more surfactants selected from the group of N-acylated amino acid and/or peptide surfactants, preferably in a concentration of 0.01 to 4% (w/w), more preferably 0.01 to 0.9% (w/w);
   (e) one or more acids in a total concentration of 0.01 to 1.5 (w/w), selected from glycolic acid, benzoic acid, citric acid, oxalic acid, succinic acid, formic acid, methanesulfonic acid and amidosulfonic acid;
   (f) one or more alcohols in a total concentration of 0.3 to 92% (w/w), preferably 0.3 to 55% (w/w), more preferably 0.3 to 46% (w/w) still more preferably 0.3 to 25% (w/w) and most preferably 12 to 19% (w/w), wherein the alcohol is preferably selected from ethanol and 2-propanol;
   (g) one or more fatty acids, preferably fatty acids other than a fatty acid used as a base of a sulfonated surfactant in the composition, preferably in a concentration of 0.1-6% (w/w);
   (h) one or more lipids, sophorolipids, such as sophorolipids commercially available as Break-Thru SF 420, preferably in a concentration of 0.002-12% (w/w) or rhamnolipids, such as rhamnolipids identified as CAS No: 4348-76-9, preferably in a concentration of 0.01-9% (w/w);
   (i) D-limonene ((R)-(+)-limonene) (CAS 5989-27-5), preferably at a concentration of 0,002-6% (w/w);
   (j) geranial (CAS: 141-27-5), neral (CAS: 106-26-3) and the mixture thereof, citral (CAS: 5392-40-5);
   (k) 3-methyl-1,3-butanediol (CAS: 2568-33-4), preferably in a concentration of 0,2 to 26% (w/w);
   (l) zwitterionic amino acids, preferably in a concentration of 0.01-6% (w/w);
   (m) sodium diisopropylnaphthalene sulphonate, preferably in a concentration of 0.01 to 1.2% (w/w);
   (n) alkenyl amber anhydride, preferably in a concentration of 0.01 to 6% (w/w);
   (o) calcium/potassium sulphite, preferably in a concentration of 0.005 to 0.35% (w/w);
   (p) SAS at a concentration of 0.02-0.63% (w/w);

(q) APG and SLES or SDS in a ratio of 1:3 to 4.2:1 APG:SLES;
(r) one or more phosphorus ester surfactants with a chain length of C8 to C18; or
(s) mixtures of any of (a) to (q).

10. Composition according to any one of claims 1 to 9, wherein the composition comprises at least three surfactants and has a dynamic surface tension of less than 29 mN/m determined 30 seconds after beginning of the measurement in the tensiometer, wherein the dynamic surface tension is determined using a tensiometer equivalent to ethanol (99.8%) and wherein the value is obtained as the mean value of 3 measurements.

11. A composition according to any one of claims 1 to 10, wherein the composition comprises at least three surfactants and has a dynamic surface tension of less than 27 mN/m determined 30 seconds after beginning of the measurement in the tensiometer, preferably less than 26 mN/m or less than 25 mN/m, and wherein the dynamic surface tension is determined using a tensiometer equivalent to ethanol (99.8%) and wherein the value is obtained as the mean of 3 measurements.

12. The composition according to any one of claims 1 to 11, wherein the composition does not contain ethoxylated compounds or compounds, wherein the ratio of surfactants with ethoxylated compounds to surfactants without ethoxylated compounds and with non-ionic effect is in the range of 4:1 to 1:5, preferably 2:1 to 1:4, and particularly preferably 1:1 to 1:4.

13. The composition according to any one of claims 1 to 12, wherein the composition comprises one or more surfactants selected from the group of N-acylated amino acid surfactants and/or N-acylated peptide surfactants having an anionic effect at the pH of the solution, in a concentration of 0.01 to 0.09% (w/w) and one or more C12 to C14 fatty acid sulphonates in a concentration of 0.01 to 0.9% (w/w).

14. The composition according to any one of claims 1 to 13, wherein the composition comprises one or more C12 to C14 fatty acid sulphonates in a concentration of 0.01 to 0.9% (w/w) and/or one or more phosphorus ester-based surfactants with a chain length of C8 to C18, in the concentration of 0.008 to 2.4% (w/w), and/or one or more surfactants selected from the group of N-acylated amino acid surfactants and/or N-acylated peptide surfactants having an anionic effect at the pH of the solution, in a concentration of 0.01 to 0.9% (w/w).

15. A method of applying a composition according to any one of claims 1 to 14 to a surface for cleaning or sterilizing the same, the method comprising steps wherein the composition is mixed from at least two containers at least 21 days before application and at the latest immediately upon application and the pH of the composition differs from the pH of at least one component by at least 0.5 to 6; preferably by 1 to 5, more preferably 1 to 2.

16. A method of sterilizing a surface, the method comprising steps wherein a composition according to any one of claims 1 to 15 is applied to a surface and wherein the application in a 4-field test after 5 minutes, preferably at least log 3 or at least log 4 in a 4-field test, achieving a reduction in Clostridioides difficile sporicidal activity of at least log 2 in a 4-field test, preferably at least log 3 or at least log 4 in a 4-field test after 5 minutes, more preferably at least 4 log levels after 4 minutes or after 2 minutes, wherein the 4-field test is performed according to EN 16615.

# EUROPEAN SEARCH REPORT

**Application Number**

EP 22 18 2034

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GB 2 181 738 A (KAO CORP) 29 April 1987 (1987-04-29) * claim 1; tables 1,3,4 * | 1-16 | INV. C11D1/83 C11D3/395 C11D3/48 C11D11/00 |
| X | GB 2 219 594 A (KAO CORP [JP]) 13 December 1989 (1989-12-13) * claim 1; example 2; tables 1,3 * | 1,3-5, 7-11,15, 16 | C11D1/12 C11D1/66 C11D1/75 A61L2/18 |
| X | JP S64 1796 A (KAO CORP) 6 January 1989 (1989-01-06) * abstract * * tables 1,2 * | 1,3-6, 10-12, 15,16 | |
| A | US 5 965 514 A (WIERENGA THOMAS JAMES [US] ET AL) 12 October 1999 (1999-10-12) * the whole document * | 1-16 | |
| E | WO 2022/136656 A2 (G1 BLUETEC GMBH [DE]) 30 June 2022 (2022-06-30) * page 9; example 4.1 * | 1-4,6,7, 9-12, 14-16 | **TECHNICAL FIELDS SEARCHED (IPC)** C11D A61L |

The present search report has been drawn up for all claims

| Place of search | Date of completion of the search | Examiner |
|---|---|---|
| The Hague | 23 November 2022 | Gault, Nathalie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 22 18 2034

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2022

| Patent document cited in search report | | Publication date | Patent family member(s) | | Publication date |
|---|---|---|---|---|---|
| GB 2181738 | A | 29-04-1987 | GB 2181738 A | | 29-04-1987 |
| | | | HK 12991 A | | 08-03-1991 |
| | | | SG 102290 G | | 14-02-1991 |
| GB 2219594 | A | 13-12-1989 | GB 2219594 A | | 13-12-1989 |
| | | | HK 40593 A | | 07-05-1993 |
| | | | JP H0524198 B2 | | 07-04-1993 |
| | | | JP H01292098 A | | 24-11-1989 |
| | | | MY 104087 A | | 30-11-1993 |
| | | | SG 14693 G | | 16-04-1993 |
| JP S641796 | A | 06-01-1989 | JP H051838 B2 | | 11-01-1993 |
| | | | JP S641796 A | | 06-01-1989 |
| US 5965514 | A | 12-10-1999 | CA 2223384 A1 | | 04-06-1998 |
| | | | US 5965514 A | | 12-10-1999 |
| WO 2022136656 | A2 | 30-06-2022 | NONE | | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *CHEMICAL ABSTRACTS,* 68585-34-2
- *CHEMICAL ABSTRACTS,* 68188-18-1
- *CHEMICAL ABSTRACTS,* 151-21-3
- *CHEMICAL ABSTRACTS,* 4348-76-9
- *CHEMICAL ABSTRACTS,* 5989-27-5
- *CHEMICAL ABSTRACTS,* 141-27-5
- *CHEMICAL ABSTRACTS,* 106-26-3
- *CHEMICAL ABSTRACTS,* 5392-40-5
- *CHEMICAL ABSTRACTS,* 2568-33-4
- *CHEMICAL ABSTRACTS,* 141464-42-87732-18-568515-73-1110615-47-9
- *CHEMICAL ABSTRACTS,* 30806228-4.
- *CHEMICAL ABSTRACTS,* 79-14-1
- *CHEMICAL ABSTRACTS,* 3088-31-1
- *CHEMICAL ABSTRACTS,* 68411-97-2
- *CHEMICAL ABSTRACTS,* 75-75-2
- *CHEMICAL ABSTRACTS,* 1310-73-2